# EUROPEAN PATENT APPLICATION

(11) **EP 4 628 044 A1**
(43) Date of publication of application: **08.10.2025**
(21) Application number: 25165890.2
(22) Date of filing: 25.03.2025
(51) Int. Cl.: A61B 90/00, A61B 17/00

(54) **EVALUATION OF SURGICAL INSTRUMENT USAGE USING SENSED MAGNETISM**

(30) Priority: 28.03.2024 US 202463571207 P
(71) Applicant: MAKO Surgical Corp., Weston, FL 33331 (US)
(72) Inventor: GSELLMAN, Lucas, Medina, 44256 (US); RUEFFER, Shane Alan, Albion, 49224 (US); O'CULL, Larry Douglas, Westfield, 46074 (US)
(74) Representative: Schott, Jakob Valentin

(57) **Abstract**

A monitoring system for a surgical instrument is provided. The surgical instrument includes a magnetic element and being configured to be subjected to a temperature-based sterilization process. The monitoring system includes a sensor configured to detect a magnetism parameter of the magnetic element. The monitoring system includes a controller in communication with the sensor. The controller is configured to evaluate the magnetism parameter, and based on the evaluation, generate an output indicative of usage of the surgical instrument.

## Description

### BACKGROUND

The subject patent application claims priority to and all the benefits of U.S. Provisional Patent Application No. 63/571,207, filed on March 28, 2024, the entire contents of each are hereby incorporated by reference.

### BACKGROUND

Before or during a surgical procedure, certain surgical instruments are sterilized or in a sterile state. During the course of a surgical procedure, the surgical instrument is exposed to body parts, tissue, blood, and the pathogens that are associated with such exposure. Some surgical instruments may be reused after a sterilization process. One example sterilization process includes placing the surgical instruments within an autoclave, wherein the surgical instruments are exposed to high temperature in order to sterilize the surgical instruments for use in a subsequent surgical procedure.

Instruments often experience wear and tear due to surgical usage and exposure to sterilization. Therefore, it is often desired to monitor how much usable life the instrument has left. Monitoring instrument usage can be a complicated and costly undertaking. Often such instruments are equipped with specialized chips or processors which communicate usage information to the main host device. However, such devices are costly and not suitable to endure high-temperature sterilization. Moreover, the usage of such conventional devices is monitored as a result of installation between the instrument and its host device. Prior techniques fail to provide a robust, consistent, and durable manner of monitoring usage of the instrument as a function of how many sterilization cycles the instrument has undergone.

### SUMMARY

This Summary introduces a selection of concepts in a simplified form that are further described in the Detailed Description below. This Summary is not intended to limit the scope of the claimed subject matter nor identify key features or essential features of the claimed subject matter.

According to a first aspect, a monitoring system for a surgical instrument is provided. The surgical instrument includes a magnetic element and being configured to be subjected to a temperature-based sterilization process. The monitoring system includes a sensor configured to detect a magnetism parameter of the magnetic element. The monitoring system includes a controller in communication with the sensor. The controller is configured to evaluate the magnetism parameter, and based on the comparison, generate an output indicative of usage of the surgical instrument.

According to a second aspect, a surgical system is provided. The surgical system includes a surgical accessory comprising a magnetic element. The surgical accessory is configured to be subjected to a temperature-based sterilization process. The surgical system further includes a surgical device configured to receive the surgical accessory. The surgical device comprises a controller and a sensor. The controller is configured to detect, with the sensor, a magnetism parameter of the magnetic element, evaluate the magnetism parameter, and based on the evaluation, generate an output to indicate usage of the surgical accessory.

According to a third aspect, a surgical device is provided. The surgical device includes a magnetic element configured to be subjected to a temperature-based sterilization process and a monitoring system comprising a sensor. The monitoring system is configured to detect, with the sensor, a magnetism parameter of the magnetic element, evaluate the magnetism parameter, and based on the evaluation, generate an output to indicate usage of the surgical device.

According to a fourth aspect, a surgical instrument configured to be repeatedly subjected to cycles of a sterilization process is provided. The surgical instrument includes a body and a magnetic element coupled to the body and configured to exhibit a magnetic field. The magnetic field degrades a predictable amount for each cycle of the sterilization process.

According to a fifth aspect, a method to generate an output to indicate usage of a surgical instrument is provided. The surgical instrument includes a magnetic element and being configured to be repeatedly subjected to a temperature-based sterilization process. The method includes utilizing a sensor to detect a magnetism parameter of the magnetic element. The method further includes, within a computerized processor, comparing the magnetism parameter to a threshold, and based upon the comparison, generating the output to indicate usage of the surgical instrument. The method may further include iteratively subjecting the surgical instrument to the temperature-based sterilization process, iteratively utilizing the sensor to detect the magnetism parameter, iteratively comparing the magnetism parameter to the threshold, and generating the output in response to identifying that the magnetism parameter has degraded past the threshold.

Any of the above aspects can be combined, in whole or in part.

Any of the above aspects can be combined, in whole or in part, with any of the following implementations:

The output indicative of usage of the surgical instrument may be configured for indicating an end-of-life of the surgical instrument. The output indicative of usage of the surgical instrument may be configured for indicating how many uses to which the instrument has been subjected. The output indicative of usage of the surgical instrument may be configured for indicating how much useful life the instrument has left.

The threshold may be selected based upon predictable degradation of the magnetism parameter per cycle of the sterilization process. The predictable degradation is based upon an autoclave sterilization process.

The magnetism parameter may be one or both of a magnetic field density or a magnetic flux.

The controller may compare the magnetism parameter to the threshold by being configured to determine an absolute measured flux value from the magnetism parameter and compare the absolute measured flux value to the threshold. The controller may compare the magnetism parameter to the threshold by being configured to determine a relative measured flux value from the magnetism parameter and compare the relative measured flux value to the threshold defined as a reference flux value.

The magnetic element may include a permanent magnet. The magnetic element may include samarium cobalt, a material that exhibits excellent predictable degradation after repeated exposure to high temperature cycles. The magnetic element may include a neodymium (NdFeB) magnet, another material that exhibits excellent predictable degradation after repeated exposure to high temperature cycles.

The sensor may be disposed within an end effector of a surgical device that is configured to receive the surgical instrument. The sensor may be a magnetometer.

The surgical accessory may be a cutting instrument or a burr guard for the cutting instrument.

Other features and advantages of the implementations of the present disclosure will be readily appreciated, as the same becomes better understood, after reading the subsequent description taken in conjunction with the accompanying drawings.

### BRIEF DESCRIPTION OF THE DRAWINGS

FIG. 1 is a perspective view of a surgical system that can be utilized with the techniques described herein, according to one example.
FIGS. 2A and 2B illustrate perspective views of a robotic system that can be utilized with the techniques described herein, according to one example.
FIG. 3 is a representation of a handheld surgical instrument that can be utilized with the techniques described herein, with certain internal components shown in phantom, according to one implementation.
FIG. 4 is a representation of a robotic end effector that can be utilized with the techniques described herein, according to one implementation.
FIG. 5 illustrates surgical instrument components that can include a magnet and sensor to implement usage detection, according to one implementation.
FIG. 6A schematically illustrates an example monitoring system including a surgical instrument including a permanent magnet configured to output an indicated usage of the surgical instrument based upon measuring a magnetic parameter of the permanent magnet, comparing the magnetism parameter to a threshold, and generating the output of indicated usage based upon the comparison.
FIG. 6B schematically illustrates an alternative monitoring system including a surgical instrument including a permanent magnet configured to output an indicated usage of the surgical instrument based upon measuring a magnetic parameter of the permanent magnet, comparing the magnetism parameter to a threshold, and generating the output of indicated usage based upon the comparison.
FIG. 6C schematically illustrates an alternative monitoring system including a surgical instrument including a permanent magnet configured to output an indicated usage of the surgical instrument based upon measuring a magnetic parameter of the permanent magnet, comparing the magnetism parameter to a threshold, and generating the output of indicated usage based upon the comparison.
FIG. 7 is a flowchart of a method of implementing a magnetic degradation usage technique, according to one example.
FIG. 8 is a flowchart of a method of implementing a magnetic degradation usage technique, according to another example.
FIG. 9 is a flowchart illustrating an example method to generate an output to indicate usage of a surgical instrument including a magnetic element and being configured to be repeatedly subjected to a temperature-based sterilization process.
FIG. 10 is a graph illustrating data related to example magnetic flux degradation of a permanent magnet over a number of cycles as a factor of temperature.
FIG. 11 is a graph illustrating data related to example magnetic field strength degradation of a permanent magnet over a number of cycles as a factor of temperature.

It will be appreciated that one or more of the implementations depicted throughout the drawings may have certain components, structural features, and/or assemblies removed, depicted schematically, and/or shown in phantom for illustrative purposes.

### DETAILED DESCRIPTION

### I. Example System Overview

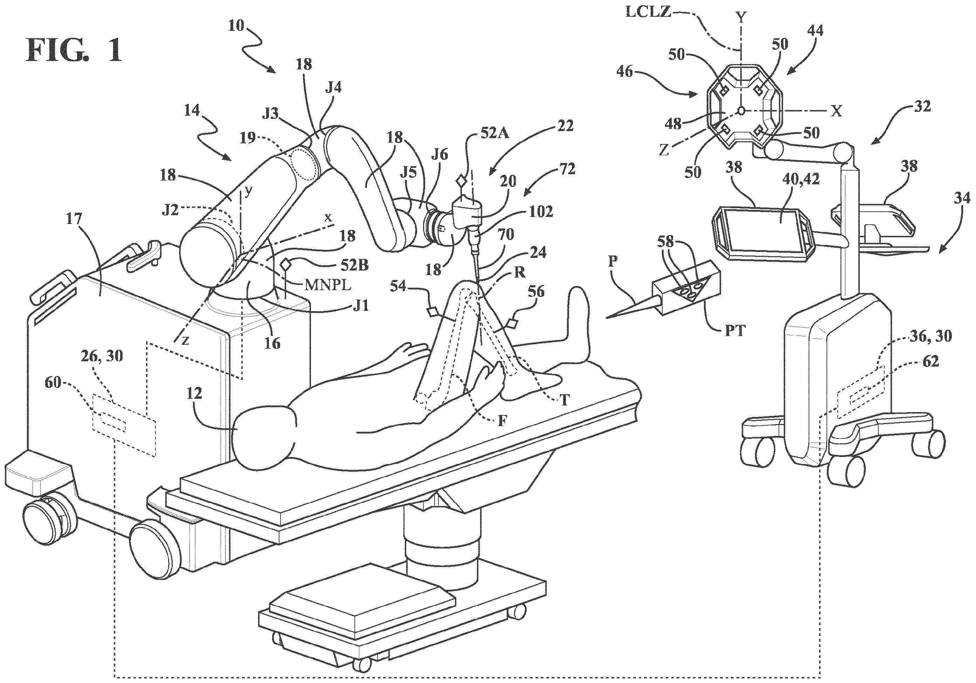

Referring to FIG. 1, a system 10 is provided. The system 10 can be a surgical system 10 adapted for treating a patient. The surgical system 10 is shown in a surgical setting such as an operating room of a medical facility. The surgical system 10 may be used to perform any intraoperative surgical procedure on a patient. Example surgical procedures include, but are not limited to: partial knee arthroplasty, total knee arthroplasty, total hip arthroplasty, shoulder arthroplasty, spinal procedures, ankle procedures, endoscopic procedures, cranial procedures, lesion removal procedures, arthroscopic procedures, arthroscopic resection procedures, soft tissue or ligament repair procedures, neurological procedures, ENT procedures, minimally invasive MIS procedures, or the like. In the example shown in FIG. 1, the patient is undergoing a knee procedure. In addition, the following implementations describe the use of the surgical system 10 in performing a procedure in which material is removed from a femur F and/or a tibia T of a patient. However, it should be recognized that the surgical system 10 may be used to perform any suitable procedure in which material is removed from any suitable portion of a patient's anatomy, material is added to any suitable portion of the patient's anatomy (e.g., an implant, graft, etc.), and/or in which any other control of and/or visualization of a surgical instrument is desired.

The system 10 may include a robotic manipulator 14. In the example shown, the robotic manipulator 14 has a base 16 and plurality of links 18. A manipulator cart 17 supports the robotic manipulator 14 such that the robotic manipulator 14 is fixed to the manipulator cart 17. The links 18 collectively form one or more arms of the robotic manipulator 14. The robotic manipulator 14 may have a serial arm configuration (as shown in FIG. 1) or a parallel arm configuration. In other examples, more than one robotic manipulator 14 may be utilized in a multiple arm configuration. The robotic manipulator 14 may comprise a plurality of (prismatic and/or rotating) joints J and a plurality of motor and/or joint encoders 19 located at the joints J for determining position data of the joints J. For simplicity, only one joint encoder 19 is illustrated in FIG. 1, although it is to be appreciated that the other joint encoders 19 may be similarly illustrated. The robotic manipulator 14 according to one example has six joints J1-J6 implementing at least six-degrees of freedom (DOF) for the robotic manipulator 14. The robotic manipulator 14 may have any number of degrees of freedom and may have any suitable number of joints J and redundant joints J.

Numerous examples of surgical instruments 20 ("instrument") will now be described. The instrument 20 can include any surgical instrument utilized in the operating room. The instrument 20 can be coupled to the manipulator 14, can be freely held in-hand, or part of any other equipment in the operating room. The instrument 20 can be a surgical device 102, a surgical accessory 70, a surgical component, or any combination 72 or sub-combination of components. The instrument 20 may be said to be aligned with instrument axis R. Examples of the instrument 20 include, but are not limited to: an end effector, a cutting tool (e.g., saw blade, cutting bur, router, reamer) that inserts into the end effector, a tool guard, a pointer P or probe, a retractor, a handheld robotic device, a cutting tool that installs to the handheld device, a navigation tracker, a mounting device (e.g., clamp, bone plate, coupling) that attaches a component to bone, a surgical implant, screws or fasteners, forceps or scissors, and the like. The instrument 20 can be any instrument that is subjected to sterilization and used in any of the described surgical procedures.

The surgical instrument 20 may couple to the robotic manipulator 14 and be movable relative to the base 16 to interact with the anatomy in certain modes. The instrument 20 is or can form part of an end effector 22. The instrument 20 may be grasped by the operator. One example arrangement of the robotic manipulator 14 and the instrument 20 is described in U.S. Patent No. 9,119,655, filed on August 2, 2013, entitled, "Surgical Manipulator Capable of Controlling a Surgical Instrument in Multiple Modes," the disclosure of which is hereby incorporated by reference. The robotic manipulator 14 and the instrument 20 may be arranged in alternative configurations. The instrument 20 can be like that shown in U.S. Patent No. 9,566,121, filed on March 15, 2014, entitled, "End Effector of a Surgical Robotic Manipulator," hereby incorporated by reference.

In some examples, the robotic manipulator 14 can be a handheld manipulator with a base portion (e.g., a portion held free-hand by the user against the force of gravity) and a moveable portion that moves relative to the base portion by actuators. The instrument 20 can be coupled to the moveable portion. Such a handheld manipulator can be like that shown in U.S. Patent No. 9,707,043, filed on August 31, 2012, entitled, "Surgical Instrument Including Housing, A Cutting Accessory that Extends from the Housing and Actuators that Establish the Position of the Cutting Accessory Relative to the Housing," and in PCT application No. PCT/US2020/042128, entitled "Robotic Hand-Held Surgical Instrument Systems and Methods," filed on July 15, 2020, the entire contents of both of which are hereby incorporated by reference.

The instrument 20 may include an energy applicator 24 designed to contact the target site, such as the tissue of a patient 12 at the surgical site. The energy applicator 24 may be a rotary cutting bur, drill, saw blade, impactor, reamer, router, ultrasonic vibrating tip, or the like.

The system 10 may include a robot system controller 30. The robot system controller 30 includes software and/or hardware for controlling the robotic manipulator 14. The robot system controller 30 directs the motion of the robotic manipulator 14 and controls a state (position and/or orientation) of the instrument 20 with respect to a coordinate system of the manipulator 14.

As shown in FIG. 1, the system 10 may further include a navigation system 32. One example of the navigation system 32 is described in U.S. Patent No. 9,008,757, filed on September 24, 2013, entitled, "Navigation System Including Optical and Non-Optical Sensors," hereby incorporated by reference. The navigation system 32 is configured to track movement of various objects. Such objects include, for example, the robotic manipulator 14, the instrument 20 and the anatomy, e.g., femur F and tibia T. The navigation system 32 tracks these objects to gather state information of each object with respect to a (navigation) localizer coordinate system LCLZ. Coordinates in the localizer coordinate system LCLZ may be transformed to a manipulator coordinate system MNPL, and/or vice-versa, using transformation techniques described herein.

The navigation system 32 may include a cart assembly 34 that houses a navigation computer 36, and/or other types of control units. A navigation interface is in operative communication with the navigation computer 36. The navigation interface includes one or more displays 38. First and second input devices 40, 42 may be used to input information into the navigation computer 36 or otherwise to select/control certain aspects of the navigation computer 36. As shown in FIG. 1, such input devices 40, 42 include interactive touchscreen displays. The input devices 40, 42 may include any one or more of a keyboard, a mouse, a microphone (voice-activation), gesture control devices, and the like. The robot system controller 30 may be implemented on any suitable device or devices in the system 10, including, but not limited to, a manipulator computer 26, the navigation computer 36, and any combination thereof. The navigation system 32 also includes a navigation localizer 44 (hereinafter "localizer") coupled to the navigation computer 36. In one example, the localizer 44 is an optical localizer and includes a camera unit 46. The camera unit 46 has an outer casing 48 that houses one or more optical sensors 50.

The navigation system 32 may include one or more trackers. In one example, the trackers include a pointer tracker PT, one or more manipulator trackers 52 (e.g., 52A, 52B), a first patient tracker 54, and a second patient tracker 56. In the illustrated example of FIG. 1, the manipulator tracker 52 is attached to the instrument 20 (*i.e*., tracker 52A), the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The pointer tracker PT is firmly affixed to a pointer P used for registering the anatomy to the localizer coordinate system LCLZ. The manipulator tracker 52 may be affixed to any suitable component of the robotic manipulator 14, in addition to, or other than the instrument 20, such as the base 16 (*i.e*., tracker 52B), or any one or more links 18 of the robotic manipulator 14. The trackers 52, 54, 56, PT may be fixed to their respective components in any suitable manner. Any one or more of the trackers may include active markers 58. The active markers 58 may include light emitting diodes (LEDs). Alternatively, the trackers 52, 54, 56 may have passive markers, such as reflectors, which reflect light emitted from the camera unit 46. Other suitable markers not specifically described herein may be utilized. The localizer 44 tracks the trackers 52, 54, 56 to determine a state of each of the trackers 52, 54, 56, which correspond to the state of the object respectively attached thereto. The localizer 44 provides the state of the trackers 52, 54, 56 to the navigation computer 36. In one example, the navigation computer 36 determines and communicates the state the trackers 52, 54, 56 to the manipulator computer 26. As used herein, the state of an object includes, but is not limited to, data that defines the position and/or orientation of the tracked object or equivalents/derivatives of the position and/or orientation. For example, the state may be a pose of the object, and may include linear data, and/or angular velocity data, and the like.

Although one example of the navigation system 32 is shown in the Figures, the navigation system 32 may have any other suitable configuration for tracking the robotic manipulator 14 and the patient 12. In one example, the navigation system 32 and/or localizer 44 are ultrasound-based. In another example, the navigation system 32 and/or localizer 44 are radio frequency (RF)-based. The navigation system 32 and/or localizer 44 may have any other suitable components or structure not specifically recited herein. Furthermore, any of the techniques, methods, and/or components described above with respect to the camera-based navigation system 32 shown throughout the Figures may be implemented or provided for any of the other examples of the navigation system 32 described herein. For example, the navigation system 32 may utilize solely inertial tracking or any combination of tracking techniques.

The robot system controller 30 further includes software modules. The software modules may be part of a computer program or programs that operate on the manipulator computer 26, navigation computer 36, or a combination thereof, to process data to assist with control of the system 10. The software modules include instructions stored in memory on the manipulator computer 26, navigation computer 36, or a combination thereof, to be executed by one or more processors of the computers 26, 36. Additionally, software modules for prompting and/or communicating with the operator may form part of the program or programs and may include instructions stored in memory on the manipulator computer 26, navigation computer 36, or a combination thereof. The operator interacts with the first and second input devices 40, 42 and the one or more displays 38 to communicate with the software modules. The user interface software may run on a separate device from the manipulator computer 26 and navigation computer 36.

The robot system controller 30 includes a manipulator controller 60 for processing data to direct motion of the robotic manipulator 14. In one example, as shown in FIG. 1, the manipulator controller is implemented on the manipulator computer 26. The manipulator controller 60 may receive and process data from a single source or multiple sources. The robot system controller 30 further includes a navigation controller 62 for communicating the state data relating to the femur F, tibia T, and robotic manipulator 14 to the manipulator controller 60. The manipulator controller 60 receives and processes the state data provided by the navigation controller 62 to direct movement of the robotic manipulator 14. In one example, as shown in FIG. 1, the navigation controller 62 is implemented on the navigation computer 36. The manipulator controller 60 or navigation controller 62 may also communicate states of the patient 12 and robotic manipulator 14 to the operator by displaying an image of the femur F and/or tibia T and the robotic manipulator 14 on the one or more displays 38. The manipulator computer 26 or navigation computer 36 may also command display of instructions or request information using the display 38 to interact with the operator and for directing the robotic manipulator 14.

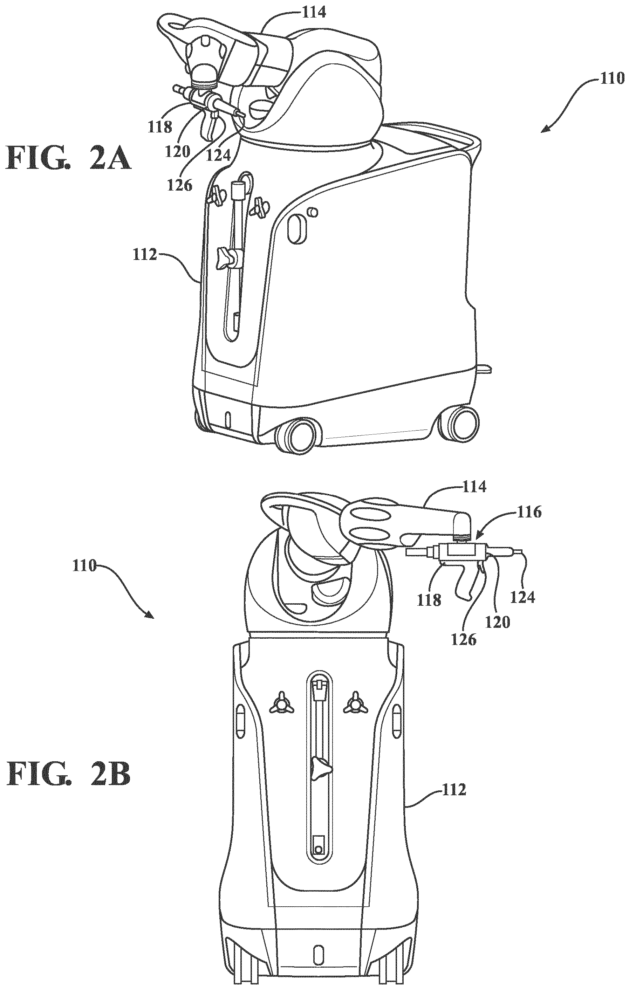

The surgical instrument 20 may be described as an instrument or an end effector capable of or configured for being utilized by a surgeon manually or semi-manually. An alternative robotic system is shown generally at 110 in FIGS. 2A and 2B. In one example, the robotic system 110 has a base 112, an arm 114, and semi-manually operable instrument 120. The instrument 120 includes an internal motor 118, a trigger actuator 126, and an instrument accessory 124. The instrument 120 may be connected to the arm 114 with a separable connection 116. The instrument 120 is semi-manual in so far as the robotic system 110 may still guide movement of the instrument 120, constrain movement of the instrument 120, or otherwise provide moderated or partially controlled operation of the instrument 120. The base 112 is mobile allowing transportation to different operating rooms, as necessary. The arm 114 is a robotic arm and is moveably attached to the base 112. The arm 114 may assist the user during a surgical procedure by supporting and aligning an end effector. The arm 114 may reduce user fatigue and error by supporting the end effector while in use, as well as restricting movement that is outside of a predetermined range. One such arm is disclosed in U.S. Patent No. 8,010,180, filed on February 21, 2006, entitled "Haptic Guidance System and Method," the disclosure of which is hereby incorporated herein by reference.

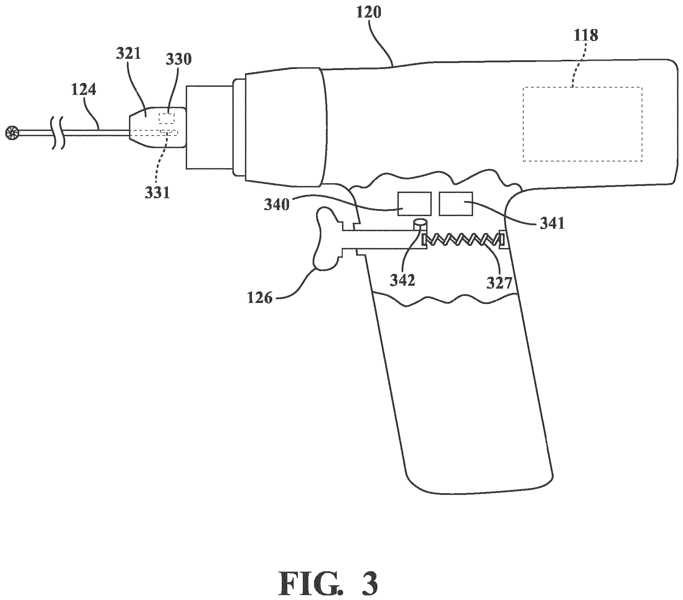

An implementation of an instrument 120 is schematically illustrated in FIG. 3. The instrument 120 may be utilized in the robotic system 110 of FIGS. 2A, 2B, wherein the instrument 120 may be operable with and supported by the arm 114. Alternatively, the instrument 120 may be handheld such that the instrument 120 is supported by a hand and arm of the user. The instrument 120 is illustrated including the trigger 126 useful for manual operation of the instrument 120 and the motor 118 configured for providing an output power to provide energy to the instrument accessory 124. A position of trigger 126 may be biased by a spring feature 327. The instrument 120 is illustrated including a receiver 321 through which instrument accessory 124 is inserted. The receiver 321 may be described as a collet, a chuck, or other similar structure. The instrument accessory 124 is illustrated including a permanent magnet 331 affixed to or formed within a shaft of the instrument accessory 124. The instrument 120 includes a corresponding magnetic signature sensor 330 positioned within the instrument 120. The magnetic signature sensor 330 may be utilized to determine a presence of or an identity of the instrument accessory 124, for example, with the instrument 120 conditionally being enabled for operation based upon a signal from the magnetic signature sensor 330 confirming that the correct instrument accessory 124 is installed to the instrument 120. Further, the trigger 126 is illustrated including a permanent magnet 342. The instrument 120 is equipped with at least two sensors 340, 341 configured to monitor proximity of and/or a position of the permanent magnet 342 relative to the sensors 340, 341.

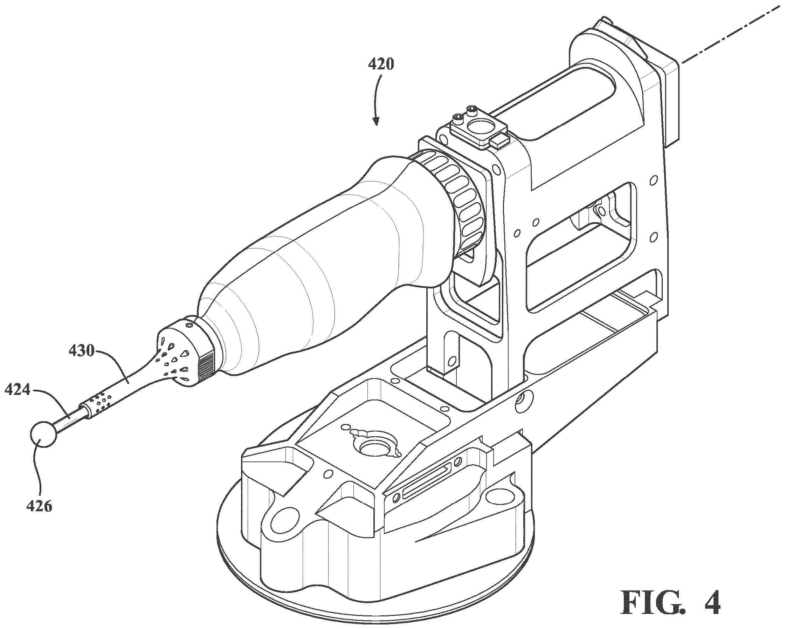

FIG. 4 illustrates an alternative implementation of an instrument 420, which may be mounted upon the robotic manipulator 14 of FIG. 1. The instrument 420 is illustrated including an instrument accessory 424 including a cutting burr 426 which may be utilized in a surgical procedure. Another instrument accessory can be a burr guard 430 surrounding a portion of the spinning shaft of the instrument accessory 424 and configured to prevent human contact to the portion of the spinning shaft.

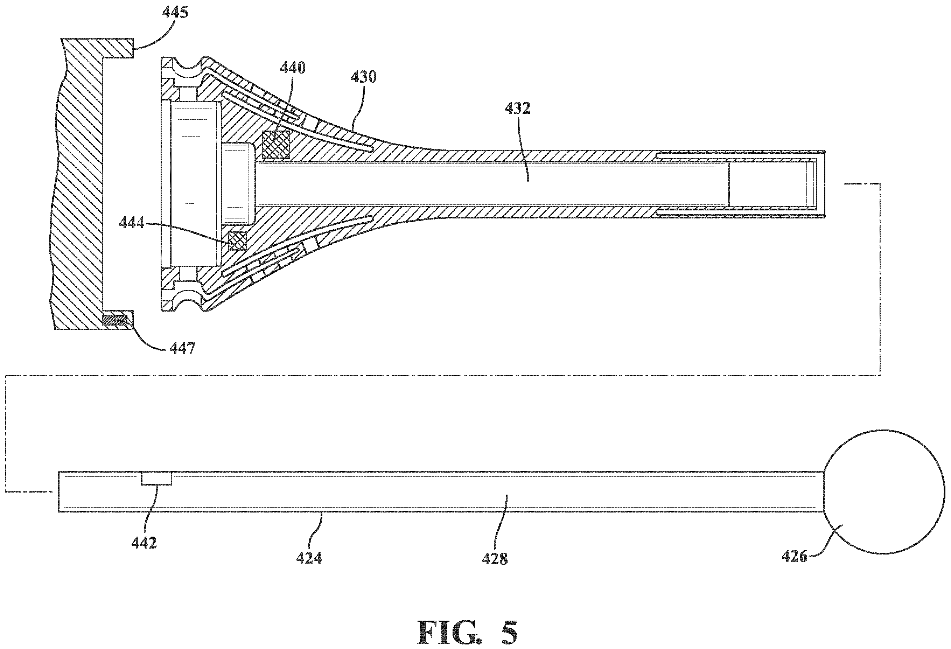

FIG. 5 illustrates the burr guard 430 and the instrument accessory 424 of FIG. 4. The burr guard 430 is illustrated in a side sectional view. The instrument accessory 424 is illustrated including the burr end 426, a shaft or body 428, and a magnetic element embodied as a first permanent magnet 442 disposed within the body 428. The burr guard 430 is illustrated including lumen 432 configured to receive the body 428, a sensor 440 configured to monitor presence of the permanent magnet 442 when the body 428 is present within the lumen 432, and a second permanent magnet 444. An end effector portion 445 is additionally illustrated including a sensor 447 configured to monitor a presence of the second permanent magnet 444. The second permanent magnet 444 may be used to confirm presence of the burr guard 430 relative to the instrument 420 of FIG. 4.

While the description and figures above provide certain examples of surgical instruments and instrument accessories, the techniques described herein can be utilized with any type of instrument or instrument accessories.

### II. Techniques for Monitoring Instrument Usage using Magnet Degradation

Described herein are various implementations of monitoring systems, surgical devices, surgical instruments, methods, and non-transitory computer readable medium comprising instructions and techniques for monitoring usage of a surgical instrument or accessory. The described techniques involve a magnetic element to be subjected to a temperature-based sterilization process and a sensor to detect a magnetism parameter of the magnetic element. The magnetism parameter is evaluated to generate an output indicative of usage of the surgical instrument/accessory.

The surgical instrument or accessory 20, 22, 120, 124, 420, 424 may each or both include a permanent magnet. Permanent magnets and sensors configured to monitor presence and properties of the magnets may be utilized in medical and surgical instruments for a number of purposes. In one implementation, the surgical instrument may include a magnetometer sensor and a magnet configured to interact with the magnetometer sensor, for example, to measure presence, identity, movement, or rotational velocity of the magnet in relation to the magnetometer sensor. A magnet may include a permanent magnet or a ferrous component that has been magnetized. A permanent magnet loses magnetism slowly over time, for example, through temperature fluctuations, presence of stray magnetic fields, and mechanical shocks, impacts, or movement. A permanent magnet may lose magnetism more rapidly if exposed to high temperatures. This loss of magnetism or this change in magnetic field properties related to a permanent magnet upon the surgical instrument or accessory 20, 22, 120, 124, 420, 424 may be used as a gage or proxy to estimate usage of the surgical instrument or accessory 20, 22, 120, 124, 420, 424. The magnet may include a magnetic element including samarium cobalt or a neodymium (NdFeB) magnet.

In one implementation, the surgical instrument or accessory 20, 22, 120, 124, 420, 424 may include a magnet useful to identify to the system 10, 110 a presence and/or an identity and/or a configuration of the surgical instrument or accessory 20, 22, 120, 124, 420, 424. A sensor such as a magnetometer sensor or a Hall Effect sensor may be utilized by the system 10, 110 to measure and indicate a presence of and a configuration of the magnet present upon the surgical instrument or accessory 20, 22, 120, 124, 420, 424.

The surgical instrument 20, 120, 420 of FIGS. 1-5, once used in a surgical procedure, may be re-used after a sterilization process is performed upon the device. Such a sterilization process may include an autoclave process, wherein the surgical instrument 20, 120, 420 may be exposed to high temperatures and liquids configured to sterilize the surgical instrument 20, 120, 420. A surgical instrument may predictably age or deteriorate after each usage and/or each corresponding operation of a sterilization process upon the surgical instrument. Actions may be taken in response to a surgical instrument reaching a certain age or going through a quantifiable amount of usage. A surgical instrument may be reconditioned, a blade may be sharpened, a replacement part may be ordered, or the surgical instrument may be retired.

A magnet includes properties which may be measured, such as magnetic field intensity (B) and magnetic flux (Φ). The magnet may deteriorate over time as a result of exposure to heat during a sterilization process or when exposed to frictional heat, such as when used as part of a rotating device. The magnet may exhibit predictable, trackable, or repeatable degradation as a result of exposure to heat. An instrument, a system, and a method useful for generating an output to indicate usage of an instrument or the instrument are provided. By tracking degradation of a permanent magnet, correlated usage of the instrument may be estimated and provided as the output. The output to indicate usage of the surgical instrument may be an estimate or indication of an age, a portion of lifespan, or a measure of accumulated usage of the surgical instrument. Wherein methods to track surgical instrument usage may include manually tracking or database tallying of how many times the surgical instrument is installed within a device or signed into a surgical procedure, a disclosed instrument, system, and method to estimate surgical instrument accumulated usage or a measure of instrument lifespan duration is provided utilizing magnetic signature degradation. The surgical instrument may include one or more of a variety of surgical instruments described above, such as any surgical instrument or accessory 20, 22, 120, 124, 420, 424 of FIGS. 1-5.

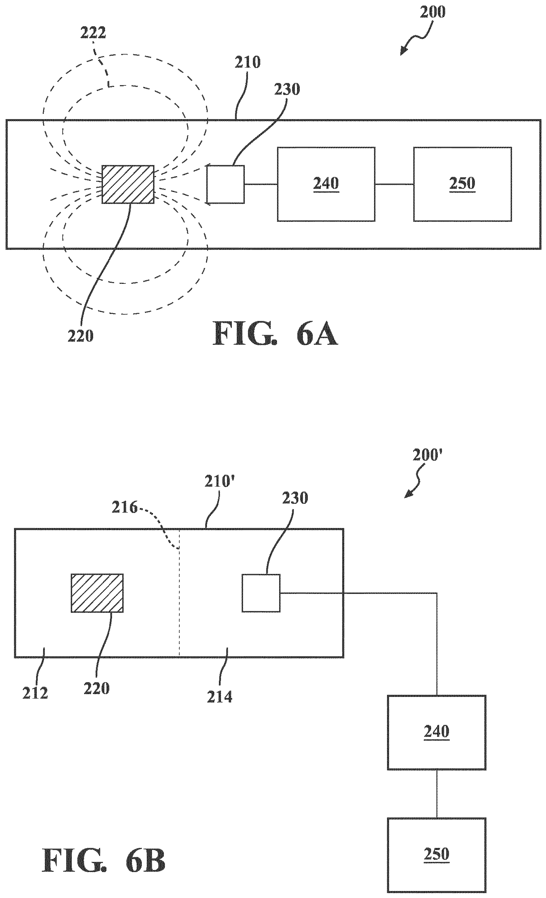

FIG. 6A schematically illustrates an example monitoring system 200 including a surgical instrument 210 including a magnetic element 220 configured to output an indicated usage of the surgical instrument 210 based upon measuring a magnetic parameter of the permanent magnet, comparing the magnetism parameter to a threshold, and generating the output of indicated usage based upon the comparison. Throughout the disclosure, magnetic element 220 may be construed to include a permanent magnet.proc The monitoring system 200 is illustrated including the surgical instrument 210 including magnetic element 220, a sensor 230 configured to measure the magnetic parameter of the magnetic element 220, and a controller 240 configured to operate programming to evaluate measurements gathered by the sensor 230. The surgical instrument 210 may be a one-piece device. The sensor 230 may additionally be utilized in operation of the surgical instrument 210, for example, for confirming presence and/or identity of the surgical instrument 210, or the sensor 230 may be specifically present for the purpose of taking measurements of a magnetic field 222 created by the magnetic element 220 for the purpose of the disclosure. Measurements taken by the sensor 230 of the magnetic field 222 generated by the magnetic element 220 may be utilized, as described herein, for estimating an indicated usage of the surgical instrument 210.

The controller 240 may be a circuit board, an integrated circuit, or a computerized device including a processor, random-access memory (RAM), and durable storage memory. The term "memory" is intended to comprise memory associated with a processor such as a CPU, and may include, for example, RAM (random access memory), ROM (read only memory), a fixed memory device (for example, hard drive), a removable memory device (for example, diskette), a flash memory, combinations thereof, and the like. The controller 240 includes programming configured to monitor measurements provided by the sensor 230, evaluate the measurements, and to estimate an indicated usage of the first portion 212 or of the surgical instrument 210 based upon the evaluation. The controller 240 may be a standalone electronic device, the controller 240 may be a part of a larger system controller or computerized unit, or the controller 240 may describe functionality spanning a plurality of physical devices. The controller 240 can receive sensor measurements using hard-wired or wireless communication. Hence, in some cases, the controller 240 can be remote from the surgical instrument.

The controller 240 may be connected to or configured to communicate with an output device 250 configured to display or take action based upon an output of indicated usage provided by the controller 240. The output device 250 may include one or more light emitting diode (LED) lights configured to emit a color of light based upon output of indicated usage provided by the controller 240, e.g., with green indicating early-lifespan period or conforming indicated usage, with yellow indicating indicated usage in a warning or mid-lifespan period, and with red indicating end-of lifespan or indicated time for refurbishment or replacement. The output device 250 may emit a tone or a vibration, transmit data to a database, or provide another type of output useful to convey the output of indicated usage. The output device 250 may be a display, such that when a user activates a scan of the surgical instrument 210, the output of indicated usage may be displayed to a user by text and/or graphic. In one example, a percentage of expected lifespan remaining may be displayed. In another example, a prompt asking the user to command ordering a replacement instrument may be presented. Such a command may be relayed to a local facility manager or directly to a medical device vendor. A visual display capable of displaying graphics may provide the output of indicated usage in a variety of ways, and the disclosure is not intended to be limited to the examples provided.

The implementation of FIG. 6A may include, in one example, the instrument accessory 124 of FIG. 3 with the magnetic element 220, the sensor 230, the controller 240, and the output device 250 each disposed upon the instrument accessory 124. The output device 250 may include circuitry to output data wirelessly to a remote computerized device or a remote server device, with the output data describing accumulated or historical usage of the instrument accessory 124 based upon monitored degradation of properties of the magnetic element 220.

FIG. 6B schematically illustrates an alternative monitoring system 200' including a surgical instrument 210' including a magnetic element 220 configured to output an indicated usage of the surgical instrument 210' based upon measuring a magnetic parameter of the permanent magnet, comparing the magnetism parameter to a threshold, and generating the output of indicated usage based upon the comparison. The monitoring system 200' is illustrated including the surgical instrument 210' including magnetic element 220. The surgical instrument 210' further includes a sensor 230 configured to measure the magnetic parameter of the magnetic element 220. The monitoring system 200' further includes a controller 240 configured to operate programming to evaluate measurements gathered by the sensor 230. The surgical instrument 210' may be a one-piece device. In the illustrated implementation of FIG. 6B, the surgical instrument 210' is illustrated including a first portion 212 and a second portion 214. In the illustrated implementation, the permanent magnet is in the first portion 212 and the sensor 230 is in the second portion 214. The first portion 212 and the second portion 214 may be separable, e.g., at parting line 216. In one example implementation, the surgical instrument 210' may include the second portion 214 embodied as a handheld trigger-grip instrument and the first portion 212 embodied as an instrument end powered, turned, or otherwise controlled by the second portion 214. The sensor 230 may additionally be utilized in operation of the surgical instrument, for example, for confirming presence and/or identity of the first portion 212, or the sensor 230 may be specifically present for the purpose of taking measurements of a magnetic field created by the magnetic element 220 for the purpose of the disclosure. Measurements taken by the sensor 230 of the magnetic field generated by the magnetic element 220 may be utilized, as described herein, for estimating an indicated usage of the first portion 212 and/or of the surgical instrument 210'.

The controller 240 and the output device 250 of FIG. 6B may be standalone devices, may be integrated together into a single device, or may be disposed within a larger electronic system, such as a robotic controller.

The implementation of FIG. 6B may include the first portion 212 embodied in one example as the instrument accessory 124 of FIG. 3 with the magnetic element 220 being disposed upon or within an instrument accessory 124. The sensor 230 may be disposed upon a connecting instrument 20, 120, 420 of FIGS. 1-5 which communicates data from the sensor 230 to the controller 240. The controller 240 and the output device 250 may each be disposed upon equipment associated with a robot or controlling surgical monitoring system or may be disposed at a remote location, for example, with a remote server device operated by a hospital, a hospital system, or a surgical equipment monitoring company. The output device 250 may include circuitry to output data visually, as data within a computer reporting program, and/or as procedure enabling/disabling protocols based upon a determined aggregated usage of the end first portion 212.

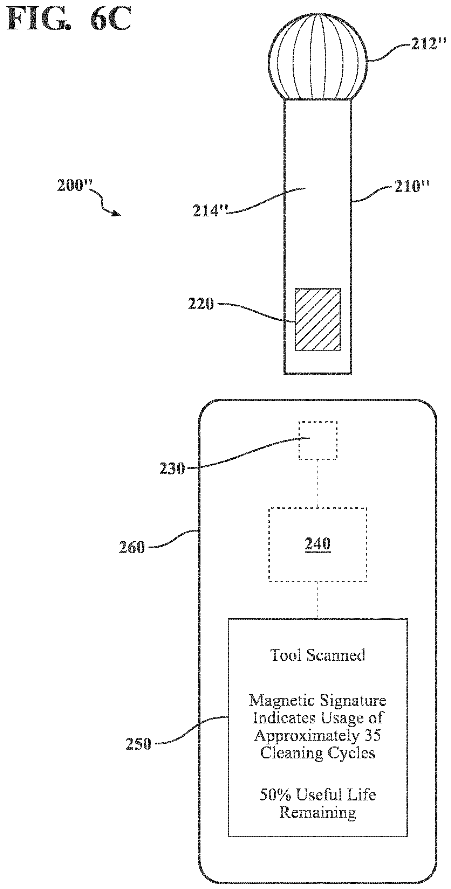

FIG. 6C schematically illustrates an alternative monitoring system 200" including a surgical instrument 210" including a magnetic element 220 configured to output an indicated usage of the surgical instrument 210" based upon measuring a magnetic parameter of the permanent magnet, comparing the magnetism parameter to a threshold, and generating the output of indicated usage based upon the comparison. The monitoring system 200" is illustrated including the surgical instrument 210" including a shaft 214" and a magnetic element 220. The monitoring system 200" is further illustrated including a magnet scanning device 260. The magnet scanning device includes a sensor 230 configured to measure the magnetic parameter of the magnetic element 220, a controller 240 configured to operate programming to evaluate measurements gathered by the sensor 230, and output device 250. The magnet scanning device 260 may include a handheld battery-powered unit including the output device 250 embodied as a display and a trigger mechanism useful for instructing the sensor 230 to record a measurement. Measurements taken by the sensor 230 of a magnetic field generated by the magnetic element 220 may be utilized, as described herein, for estimating an indicated usage of a first portion 212" and/or of the surgical instrument 210". The magnet scanning device 260 may be a standalone unit or may be incorporated within a wider instrument management system, for example, tracking and recording details about various instruments within a facility or a procedure management system, such as checking the output of indicative usage as part of a pre-op or post-op checklist. In another example, the surgical instrument 210" may be one of many surgical instruments 210" in a storage room, and the magnet scanning device 260 may be periodically utilized to check the inventory in the storage room to assess which require maintenance or replacement.

A wide variety of graphic outputs may be used to convey information to a user regarding an indicated usage of a surgical instrument. In the example of output device 250, a display screen configured to display text may indicate "Instrument Scanned," providing confirmation to the user that the surgical instrument was successfully evaluated by the sensor 230 and the controller 240. The display screen may further display text to indicate "Magnetic Signature Indicates Usage of Approximately 35 Cleaning Cycles," indicating a measure of accumulated or historical usage of the surgical instrument. This value may be described as a number of cleanings, a number of surgical procedures, an estimated elapsed time used in surgery, or other similar metrics. The display screen may further display text to indicate "50% Useful Life Remaining," providing the user an estimate or evaluation of how much useful life is left in the surgical instrument. Other outputs may be generated, for example, including graphical outputs which may be color based (e.g., green indicating more than 25% of useful life remaining in the surgical instrument; yellow indicating less than 25% of useful life remaining in the surgical instrument, but that the instrument has not yet reached end of life; and red indicating that the surgical instrument has reached end of life.) In another example, a pie graph or a bar graph metric may be used to convey to the user how much of the useful life of the surgical instrument has been used and how much remains. In another embodiment, data may be transferred to a remote server device or a computerized device configured to manage surgical instruments of a particular type, for a particular operating room or hospital floor, or for other particular uses, and a computerized program or application may be operated to tabulate or accumulate data related to a status of a plurality of surgical instruments within the facility.

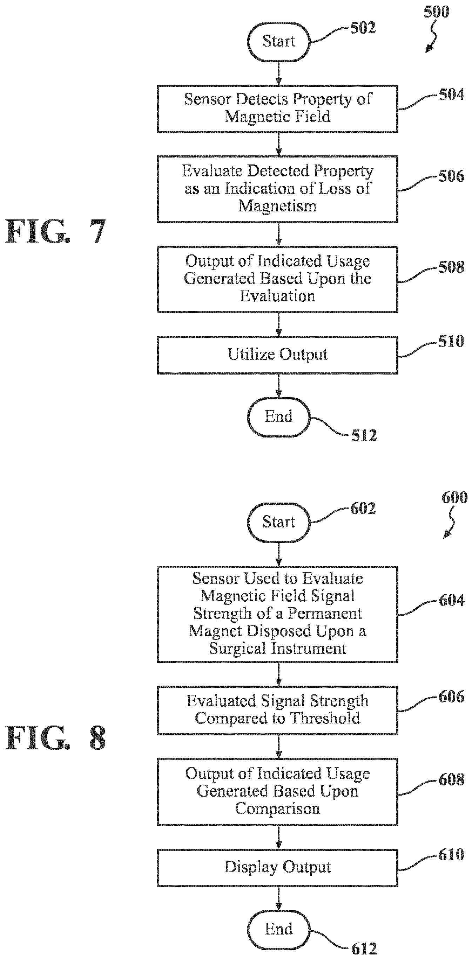

FIG. 7 is a flowchart illustrating an example method 500 to generate an output to indicate usage of a surgical instrument including a magnetic element and being configured to be repeatedly subjected to a temperature-based sterilization process. The method 500 is described being operated including monitoring systems 200, 200', 200" with surgical instruments 210, 210' 210" of FIGS. 6A-6C, although the method 500 may be operated with the implementations of FIGS. 1-5 or other variations thereof. The method 500 starts at step 502. At step 504, a sensor 230 detects a property of magnetic field of a magnetic element 220, for example, including one of a magnetic field strength or a magnetic flux. At step 506, the detected property is evaluated to determine whether a loss of magnetism in magnetic element 220 is indicated. Such a loss in magnetism may be generic to the magnetic element 220, for example, with "new" magnets 220 of a particular specification being assigned a nominal magnetism property initial value, and the detected value from step 504 may be compared to the nominal initial value. In another example, the particular magnetic element 220 may be registered or saved as a specific entity, and subsequent iterations of detection of the magnetic field of the particular magnetic element 220 may be compared to identify a trend in the loss in of magnetism for that particular magnetic element 220. A number of evaluation methods to determine loss of magnetism are envisioned, and the disclosure is not intended to be limited to the examples provided herein. At step 508, an output of indicated usage is generated based upon the evaluation. At step 510, the output of indicated usage is utilized in a tangible, real-world output that enables tracking of usage information, refurbishment, or repair of the surgical instrument 210, 210', 210", or disposition and replacement of the surgical instrument 210, 210', 210". At step 512, the method 500 ends. The method 500 is provided as an example of a method for generating an output to indicate usage of a surgical instrument including a magnetic element based upon degradation of properties of a magnetic field generated by the magnetic element after the magnetic element has been subjected to a plurality of high-temperature cycles. A number of additional and/or alternative method steps are envisioned, and the disclosure is not intended to be limited to the examples provided herein.

FIG. 8 is a flowchart illustrating an example method 600 to generate an output to indicate usage of a surgical instrument including a magnetic element and being configured to be repeatedly subjected to a temperature-based sterilization process. The method 600 is described being operated including monitoring systems 200, 200', 200" with surgical instruments 210, 210' 210" of FIGS. 6A-6C, although the method 600 may be operated with the implementations of FIGS. 1-5 or other variations thereof. The method 600 may be described as an alternative implementation to the method 500 of FIG. 7. The method 600 starts at step 602. At step 604, a sensor 230 is used to evaluate a magnetic field signal strength of a magnetic element 220, for example, including one of a magnetic field strength or a magnetic flux, wherein the magnetic element 220 is a permanent magnet disposed upon a surgical instrument. At step 606, the evaluated signal strength is compared to a threshold value. The comparison may include evaluation of relative values of magnetic flux or magnetic field strength of the magnetic element 220. For example, an original value of a magnetic field property of magnetic element 220 with each new surgical instrument 210, 210', 210" may be measured at a time when the surgical instrument 210, 210', 210" is new. Subsequent values of the magnetic field property may be measured after the surgical instrument 210, 210', 210" undergoes usage and sterilization cycles. The identity of the surgical instrument 210, 210', 210" and the corresponding magnetic element 220 may be tracked. An indicated usage may be determined based upon a relative threshold drop in values from the original values to the subsequent values. In the alternative, the comparison may include evaluation of determined or measured magnetic flux or magnetic field strength to absolute or preset/selected values. An original or new magnetic field property value may be preset based upon historical or statistical values for similar magnets 220. An absolute value of a magnetic field property may be selected to indicate a particular indicated usage. When the measured magnetic field property drops below the absolute value selected as the threshold, the surgical instrument may be stated to have undergone a corresponding indicated usage. Degradation of the property of the magnetic field may be utilized as described herein to estimate or generate an output of indicated usage for the surgical instrument 210, 210', 210" associated with the magnetic element 220. At step 608, an output of indicated usage is generated based upon the comparison. At step 610, the output of indicated usage is displayed, conveying information to a viewer relative to the estimated usage of the surgical instrument. At step 612, the method 600 ends. The method 600 is provided as an additional example of a method for generating an output to indicate usage of a surgical instrument including a magnetic element based upon degradation of properties of a magnetic field generated by the magnetic element after the magnetic element has been subjected to a plurality of high-temperature cycles. A number of additional and/or alternative method steps are envisioned, and the disclosure is not intended to be limited to the examples provided herein.

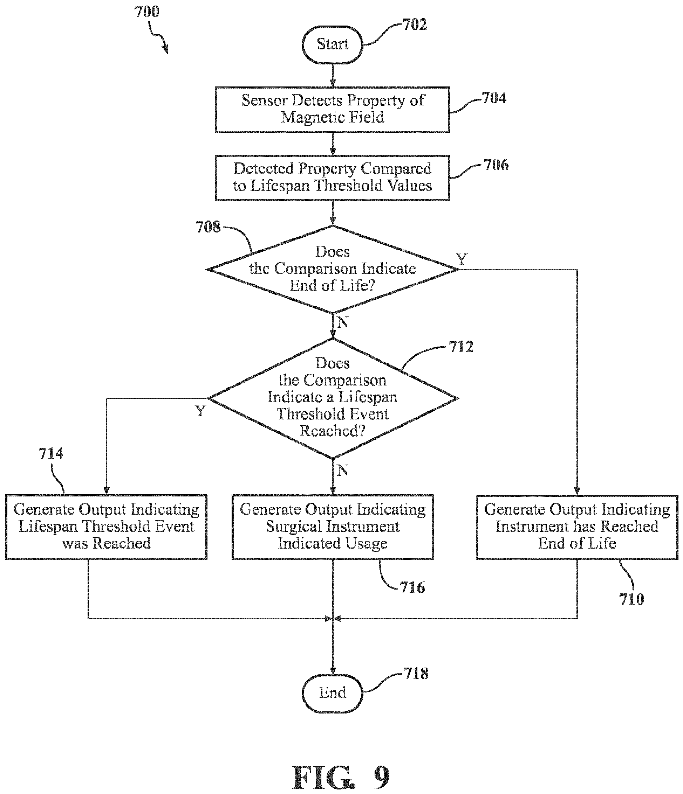

FIG. 9 is a flowchart illustrating an example method 700 to generate an output to indicate usage of a surgical instrument including a magnetic element and being configured to be repeatedly subjected to a temperature-based sterilization process. The method 700 is described being operated including monitoring systems 200, 200', 200" with surgical instruments 210, 210' 210" of FIGS. 6A-6C, although the method 700 may be operated with the implementations of FIGS. 1-5 or other variations thereof. The method 700 may be described as an alternative implementation to the method 500 of FIG. 7. The method 700 starts at step 702. At step 704, a sensor 230 is used to detect a property of a magnetic field of a magnetic element 220, for example, including one of a magnetic field strength or a magnetic flux, wherein the magnetic element 220 is a permanent magnet disposed upon a surgical instrument. At step 706, the detected property is compared to one or more lifespan threshold values or reference property values selected or determined to correspond to lifespan threshold events such as selected threshold amounts of indicated usage of a surgical instrument. Examples may include a lifespan threshold value corresponding to an end of life estimate for the surgical instrument, a lifespan threshold value corresponding to a percentage lifetime remaining in the surgical instrument (e.g. corresponding to the instrument having 50% or 25% of useful life remaining/time to order a new instrument), a lifespan threshold value corresponding to an estimated drop in effectiveness (e.g. corresponding to the instrument likely being dulled to a point that instrument movement speeds should be reduced), or other similar threshold indications. At step 708, a determination is made whether the comparison indicates an end of useful life for the surgical instrument. If the determination is made that the end of useful life for the surgical instrument is indicated, the method 700 advances to step 710, wherein an output is generated indicating that the instrument has reached end of life. If at the step 708, a determination is made that the end of useful life for the surgical instrument is not indicated, the method 700 advances to step 712. At step 712, a determination is made whether the comparison indicates that a lifespan threshold event has been reached. If the determination is made that a lifespan threshold event has been reached, the method 700 advances to step 714, wherein an output is generated indicating that a lifespan threshold event was reached. If, at step 712, the determination is made that a lifespan threshold event has not been reached, the method 700 advances to step 716, wherein an output indicating surgical instrument indicated usage may be generated. After an output is generated at one of the steps 710, 714, and 716, the method 700 advances to step 718, wherein the method 700 ends. The method 700 is provided as an additional example of a method for generating an output to indicate usage of a surgical instrument including a magnetic element based upon degradation of properties of a magnetic field generated by the magnetic element after the magnetic element has been subjected to a plurality of high-temperature cycles. A number of additional and/or alternative method steps are envisioned, and the disclosure is not intended to be limited to the examples provided herein.

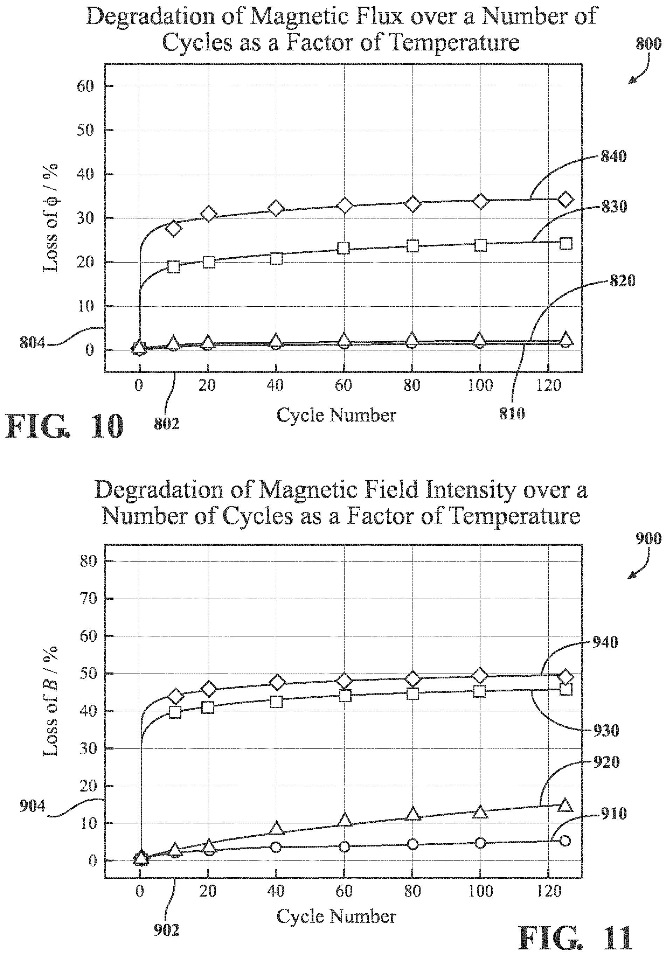

FIGS. 10 and 11 include data regarding degradation of example magnets when exposed to cycles of high temperature. FIGS. 10 and 11 are published as "Figure 2" in H.-L. Wu, Z.-M. Long, K.-Q. Song, C.-Q. Li, D.-L. Cong, B. Shao, X.-W. Liu, Y.-L. Ma. The Effect of Temperature Cycling on the Magnetic Degradation and Microstructure of a Zn-Coated NdFeB Magnet. Coatings 2022, 12, 660. https://doi.org/10.3390/coatings12050660. FIG. 10 is a graph 800 illustrating data related to example magnetic flux degradation of a permanent magnet over a number of cycles as a factor of temperature. The graph 800 includes a horizontal axis 802 describing a number of high temperature exposures to which a permanent magnet is exposed. The graph 800 further includes a vertical axis 804 describing a loss of magnetic flux as a percentage of the original magnetic flux of the permanent magnet being tested. Test data plots 810, 820, 830, 840 are provided including a plurality of respective temperature exposure ranges, including the test data plot 810 representing a magnet exposed to temperatures up to 120°C, the test data plot 820 representing a magnet exposed to temperatures up to 140°C, the test data plot 830 representing a magnet exposed to temperatures up to 160°C, and the test data plot 840 representing a magnet exposed to temperatures up to 180°C. One may see from the test data plots 810, 820, 830, 840, particularly when temperature exposure is over or within a particular or threshold temperature range, that magnetic flux is predictably degraded over increasing temperature cycle counts.

FIG. 11 is a graph 900 illustrating data related to example magnetic field strength degradation of a permanent magnet over a number of cycles as a factor of temperature. The graph 900 includes a horizontal axis 902 describing a number of high temperature exposures to which a permanent magnet is exposed. The graph 900 further includes a vertical axis 904 describing a loss of magnetic field strength as a percentage of the original magnetic field strength of the permanent magnet being tested. Test data plots 910, 920, 930, 940 are provided including a plurality of respective temperature exposure ranges, including the test data plot 910 representing a magnet exposed to temperatures up to 120°C, the test data plot 920 representing a magnet exposed to temperatures up to 140°C, the test data plot 930 representing a magnet exposed to temperatures up to 160°C, and the test data 940 representing a magnet exposed to temperatures up to 180°C. One may see from the test data plots 910, 920, 930, 940, particularly when temperature exposure is over or within a particular or threshold temperature range, that magnetic field strength is predictably degraded over increasing temperature cycle counts.

As will be appreciated by one skilled in the art, the implementations described herein may include a computer program product embodied in one or more computer readable medium(s) having computer readable program code embodied thereon. Computer software including instructions or code for performing the methods described herein, may be stored in one or more of the associated memory devices (for example, ROM, fixed or removable memory) and, when ready to be utilized, loaded in part or in whole (for example, into RAM) and implemented by a CPU. Such software could include, but is not limited to, firmware, resident software, microcode, and the like.

Several embodiments have been described in the foregoing description. The embodiments discussed herein are not intended to be exhaustive or limit to any particular form. The terminology, which has been used, is intended to be in the nature of words of description rather than of limitation. Many modifications and variations are possible in light of the above teachings and the invention may be practiced otherwise than as specifically described. Furthermore, the headings used in this document are introduced solely for reference and readability purposes and should not be understood to limit the content of the section solely by the subject matter of the heading.

## Claims

1. A monitoring system (200, 200', 200") for a surgical instrument (210, 210', 210"), the surgical instrument (210, 210', 210") comprising a magnetic element (220) and being configured to be subjected to a temperature-based sterilization process, the monitoring system (200, 200', 200") comprising:
a sensor (230, 330, 440, 447) configured to detect a magnetism parameter of the magnetic element (220); and
a controller (240) in communication with the sensor (230, 330, 440, 447) and configured to:
evaluate the magnetism parameter, and
based on the evaluation, generate an output indicative of usage of the surgical instrument (210, 210', 210").

2. The monitoring system of claim 1, wherein the output indicative of usage of the surgical instrument (210, 210', 210") is configured for indicating an end-of-life of the surgical instrument (210, 210', 210").

3. The monitoring system of claim 1, wherein the output indicative of usage of the surgical instrument (210, 210', 210") is configured for indicating:
how many uses to which the surgical instrument (210, 210', 210") has been subjected; and/or
how much useful life the surgical instrument (210, 210', 210") has left.

4. The monitoring system (200, 200', 200") of claim 1, further comprising the controller (240) configured to evaluate the magnetism parameter by comparing the magnetism parameter relative to a threshold; and
wherein the threshold is selected based upon a predictable degradation of the magnetism parameter per cycle of the sterilization process.

5. The monitoring system (200, 200', 200") of claim 4, wherein the predictable degradation is based upon an effect upon the magnetic element (220) due to an autoclave sterilization process.

6. The monitoring system (200, 200', 200") of claim 4, wherein the controller (240) compares the magnetism parameter to the threshold by comparing the magnetism parameter to:
an absolute threshold value; or
previously recorded magnetism parameter values and determining whether a relative threshold drop has occurred based upon the comparing.

7. The monitoring system (200, 200', 200") of claim 1, wherein the magnetism parameter is one or both of a magnetic field density or a magnetic flux.

8. The monitoring system (200, 200', 200") of claim 1, wherein the magnetic element (220) includes samarium cobalt.

9. The monitoring system (200, 200', 200") of claim 1, wherein the magnetic element (220) includes a neodymium (NdFeB) magnet.

10. The monitoring system (200, 200', 200") of claim 1, wherein the sensor (230, 330, 440, 447) is disposed within an end effector (22) of a surgical device (102) that is configured to receive the surgical instrument (210, 210', 210").

11. The monitoring system (200, 200', 200") of claim 1, wherein the sensor (230, 330, 440, 447) is a magnetometer.

12. A surgical system (10) comprising:
the surgical instrument (210, 210', 210") comprising the magnetic element (220) of claim 1; and
a surgical device (102) configured to receive the surgical instrument (210, 210', 210"), wherein the surgical device (102) comprises the monitoring system (200, 200', 200") of claim 1.

13. A surgical device (102) comprising:
the magnetic element (220) of claim 1; and
the monitoring system (200, 200', 200") of claim 1.

14. The surgical instrument (210, 210', 210") of claim 1, further comprising:
a body (428); and
the magnetic element (220) coupled to the body (428) and configured to exhibit a magnetic field (222); and
wherein the magnetic field (222) degrades a predictable amount for each cycle of the sterilization process.

15. A method of operating the monitoring system (200, 200', 200") of claim 1 to generate the output to indicate usage of the surgical instrument (210, 210', 210").
